(19) Europäisches Patentamt
European Patent Office
Office européen des brevets

(11) Veröffentlichungsnummer: **0 014 767**
**B1**

(12) **EUROPÄISCHE PATENTSCHRIFT**

(45) Veröffentlichungstag der Patentschrift: **05.05.82**

(51) Int. Cl.³: **C 07 C 127/22** // C08F20/60, C02F1/54, D21H3/38

(21) Anmeldenummer: **79105339.0**

(22) Anmeldetag: **21.12.79**

(54) **Quartäre Gruppen enthaltende Acrylyl- bzw. Methacrylylharnstoffe und Verfahren zu ihrer Herstellung.**

(30) Priorität: **27.12.78 DE 2856384**

(43) Veröffentlichungstag der Anmeldung: **03.09.80 Patentblatt 80/18**

(45) Bekanntmachung des Hinweises auf die Patenterteilung: **05.05.82 Patentblatt 82/18**

(84) Benannte Vertragsstaaten: **AT BE CH DE FR GB IT LU NL SE**

(56) Entgegenhaltungen: **FR-A-2 259 816**

(73) Patentinhaber: **Chemische Fabrik Stockhausen & Cie., Bäkerpfad 25, D-4150 Krefeld (DE)**

(72) Erfinder: **Küster, Erich, Dipl.-Chem. Dr., Alexanderplatz 15, D-4150 Krefeld (DE)**
Erfinder: **Dahmen, Kurt, Dipl.-Chem., Dr., Von-Velsen-Strasse 6, D-4050 Mönchengladbach 2 (DE)**
Erfinder: **Barthell, Eduard, Dipl.-Chem., Dr., Minkweg 18a, D-4150 Krefeld (DE)**

(74) Vertreter: **Klöpsch, Gerald, Dr., An Gross St. Martin 6, D-5000 Köln 1 (DE)**

EP 0 014 767 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Quartäre Gruppen enthaltende Acrylyl- bzw. Methacrylylharnstoffe und Verfahren zu ihrer Herstellung

Die Erfindung betrifft quartäre Gruppen enthaltende Acrylyl- bzw. Methacrylylharnstoffe und Verfahren zu ihrer Herstellung. Die erfindungsgemässen Harnstoffe können zur Herstellung von Polymerisaten verwendet werden.

Praktisch alle kationischen Polymeren und die Monomeren, aus denen sie hergestellt werden, leiten ihre kationischen Merkmale von quartären Amoniumgruppen her (Literaturübersicht bei M.F. Hoover, «J. Macromol. Sci. Chem.» A 4 *6* (1970), S. 1327-1418). Im allgemeinen stellt man zunächst das tertiäre Amin (z.B. 2-(Dimethylamino)äthylmethacrylat oder N-(3-Dimethylaminopropyl)acrylamid her, das anschliessend mit einem geeigneten Reagens quarterniert wird.

Im Gegensatz hierzu wird in der vorliegenden Erfindung ein tertiäres Amin mit einer olefinisch ungesättigten Halogenverbindung direkt zu kationischen Monomeren alkyliert.

Gegenstand der Erfindung sind daher Harnstoffe der allgemeinen Formel (I)

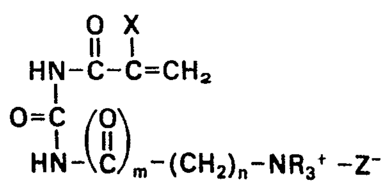

(I)

in der X entweder Wasserstoff oder eine Methylgruppe, R ein niederer Alkylrest mit 1 bis 4 Kohlenstoffatomen ist, m entweder 0 oder 1, n eine ganze Zahl zwischen 1 und 4 ist und Z ein beliebiges salzbildendes Anion bedeutet.

Die Verbindungen der Formel I werden durch Umsetzung von halogensubstituierten Harnstoffen der allgemeinen Formel (II)

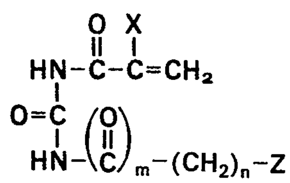

(II)

in der X, m und n die oben angegebenen Bedeutungen haben und Z Halogen, insbesondere Chlor, Brom oder Jod ist, mit Trialkylaminen nach bekannten Methoden (J. Goerdeler in Houben-Weyl, «Methoden der organischen Chemie», Band 11/2, S.592 ff., Georg Thieme Verlag, Stuttgart, 4.Aufl. 1958) erhalten. Die Quarternierung wird in einem Lösungsmittel durchgeführt. Als Lösungsmittel dienen bevorzugt aliphatische und aromatische Kohlenwasserstoffe, Alkohole und/oder Ketone.

Die Reaktionstemperatur liegt im Bereich von 20 bis 100°C; sie richtet sich in erster Linie nach dem Halogensubstituenten im Ausgangsmaterial. Während die Jodverbindung schon bei Raumtemperatur reagiert, benötigen Chlor- und Bromverbindungen erhöhte Temperaturen. Zweckmässigerweie arbeitet man dann unter einem Druck von bis zu 4 bar. Man erhält die quartären Substanzen in fester Form als weisse, leicht wasserlösliche Pulver. Gemäss einer besonders bevorzugten Ausführungsform findet die Reaktion bei 20 bis 100°C in Azeton als Lösungsmittel und mit Trimethylamin als tertiärem Amin statt.

Das Anion ist nicht auf die Klasse der Halogenide beschränkt. Andere Formen können leicht durch Verdrängungsreaktionen oder durch Ionenaustauschprozess eingeführt werden, so dass z.B. die Sulfate, Perchlorate, Nitrate oder Acetate erhalten werden.

Die Herstellung der halogensubstituierten Harnstoffe gemäss Formel II erfolgt durch Addition von Halogenalkyl- bzw. Halogenacylisocyanaten der Formel

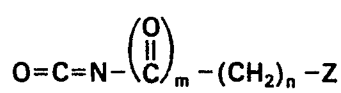

(III)

wobei m und n die oben angegebene Bedeutung haben und Z ein Halogenatom, vorzugsweise Chlor, Brom oder Jod ist, an α,β-ungesättigten Carbonsäureamide der Formel (IV)

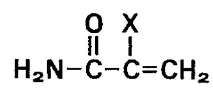

(IV)

worin X die oben angegebene Bedeutung hat. Besonders geeignet sind die ω-Halogenalkyl- und -acylisocyanate; speziell seien hier 2-Halogenäthyl-, 3-Halogenpropyl-, 4-Halogenbutyl- sowie 2-Halogenacetyl- und 3-Halogenpropionylisocyanat genannt.

Ein Gelingen dieser Reaktion war nicht von vornherein zu erwarten. Zwar ist die Umsetzung von Carbonsäureamiden mit Isocyanaten schon beschrieben [B. Kuhn, «Ber. dtsch. chem. Ges.» *17*, 2880 (1884); P.F. Wiley, «J, Amer. Chem. Soc.» *71*, 1310 (1949)], auch wurde Acrylamid nach DT-PS Nr. 888316 schon mit Arylisocyanaten umgesetzt. In der genannten DT-PS wird jedoch ausdrücklich vermerkt, dass eine Reaktion mit aliphatischen Isocyanaten nicht beobachtet wurde, so dass der glatte Verlauf dieser Additionsreaktion um so überraschender erschien.

Man erhält die Additionsprodukte der allgemeinen Formel II durch einfaches Erhitzen der beiden Reaktionspartner mit katalytischen Mengen eines tertiären Amins. Zweckmässigerweise arbeitet man unter Zusatz eines aromatischen Kohlenwasserstoffs oder hochsiedenden Äthers als Verdünnungsmittel, aus dem die Harnstoffe nach Beendigung der Reaktionszeit meist kristallin ausfallen.

Die Reaktion mit den Halogenalkylisocyanaten (m=0) führt man bevorzugt in aromatischen Kohlenwasserstoffen, z.B. Benzol, Toluol oder Xylol bei Temperaturen bis zu 140°C und Drucken von 1 bis 4 bar durch, während bei den Halogenacylisocyanaten (m=1) bevorzugt hochsiedende Äther wie 1,2-Dimethoxiäthan oder Tetrahydrofuran als Lösungsmittel verwendet werden; die Reaktion selbst läuft dann schon bei Raumtemperatur ab. Als tertiäres Amin eignen sich Trialkylamine wie Trimethylamin, Triäthylamin, Tripropylamin, Tributylamin oder auch heterocyclische Amine wie Pyridin. Bevorzugt wird Trimethylamin eingesetzt.

Ferner ist empfehlenswert, wegen der bekannten Polymerisationsfreudigkeit der (Meth)Acrylamide einen Polymerisationsverzögerer, wie z.B. Hydrochinon, Hydrochinonmonomethyläther oder Phenothiazin zuzusetzen.

Die ungesättigten Harnstoffverbindungen der Formel (I) lassen sich entweder allein oder mit anderen polymerisierbaren Monomeren polymerisieren. Der Ausdruck Polymer bedeutet Homopolymerisate, Copolymerisate, Terpolymerisate und andere Interpolymerisate. Die Polymerisate enthalten Strukturelemente der allgemeinen Formel (V), die sich von den Harnstoffen I ableiten

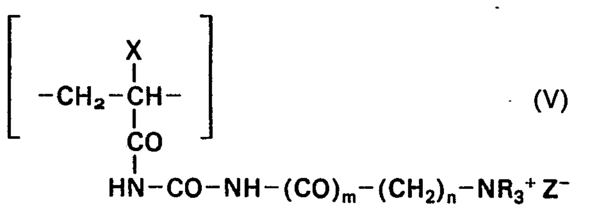

(V)

wobei X, R, m, n und Z die bei Formel (I) angegebene Bedeutung haben. Als geeignete Comonomere kommen alle wasserlöslichen, olefinisch ungesättigten Verbindungen infrage, beispielsweise Acryl- und Methacrylsäureamid und deren N-substituierte Derivate, insbesondere N-[3-Dimethylaminopropyl]-acrylamid sowie dessen Salze und quartäre Verbindungen, N-Methylolacrylamid und -methacrylamid sowie deren Äther, aminosubstituierte Acryl- und Methacrylsäureester, wie z.B. Diäthylaminoäthylacrylat und Dimethylaminoäthylmethacrylat sowie deren Salze und quartäre Verbindungen, weiterhin Vinylester, wie z.B. Vinylacetat. Entsprechende Copolymere weisen 4 bis 96 Gew.% der oben wiederkehrenden Einheiten auf, während der Rest aus einem oder mehreren damit copolymerisierbaren olefinisch ungesättigten Verbindungen besteht.

Die Polymerisation erfolgt nach bekannten Verfahren: Sie kann thermisch, photochemisch, mit Strahlen oder mit den üblichen radikalischen Initiatoren ausgelöst werden. Hierzu kann die Polymerisation in Lösung, Suspension oder Emulsion durchgeführt werden. Geeignete Initiatoren sind u.a. anorganische Peroxide, wie z.B. Wasserstoffperoxid oder organische Hydroperoxide und Peroxide, wie z.B. tert.-Butyl-, Kumolhydroperoxid oder Dibenzoylperoxid, in Radikale zerfallende aliphatische Azoverbindungen, wie z.B. 2,2'Azobis-bis-isobuttersäurenitril, Redoxkatalysatorsysteme wie die Systeme Persulfat oder Chlorat mit Disulfit oder Eisen-(II)-Salzen, ferner die als Radikalbildner bekannten Chelate von Übergangsmetallen. Die Initiatoren werden im allgemeinen in einer Menge von 0,001 bis 1 Gew.% bezogen auf die Monomermenge, verwendet. Die optimale Menge und der optimal wirksame Initiator lassen sich durch Versuche leicht ermitteln.

Die Polymerisation wird vorteilhaft in Gegenwart eines Lösungs- oder Verdünnungsmittels durchgeführt. Die bei anderen Monomeren üblichen Suspensions-, Lösungs- oder Emulsionspolimerisationsverfahren sind auch für die erfindungsgemässen Polymerisate geeignet. Gegebenenfalls können Hilfsmittel, wie Puffersubstanzen, Dispergiermittel, Schutzkolloide und dergleichen verwendet werden. Die Polymerisate können als Flockungs-, Sedimentations-, Entwässerungs- und Retentionshilfsmittel eingesetzt werden. Sie zeichnen sich durch Löslichkeit in Wasser aus. Weiterhin weisen diese wässerigen Lösungen nur geringe Viskositäten auf, was für die Anwendung sehr vorteilhaft ist. Wegen des Vorhandenseins der sehr stabilen Harnstoffgruppierungen in den Polymeren ist auch die Hydrolysebeständigkeit ausgezeichnet.

Polymerisate der erfindungsgemässen Acrylyl- und Methacrylylharnstoffe und die Verwendung der Polymerisate als Flockungs-, Sedimentations-, Entwässerungs- und Retentionshilfsmittel sind Gegenstand der Teilanmeldung EP 81104251.4.

Die Erfindung wird durch die nachfolgenden Beispiele 1 bis 9 erläutert:

A) Herstellung von Harnstoffen der Formel II:

*Beispiel 1: N-Acrylyl-N'-(2-chloräthyl)-harnstoff*

Ein Gemisch aus 71,1 g (1,0 Mol) Acrylamid, 0,5 g Triäthylamin, 0,5 g 2,6-Di-tert.-butyl-4-methylphenol und 250 ml Benzol wird mit 110,8 g (1,05 Mol) 2-Chloräthylisocyanat versetzt und 10 h in einem Autoklaven auf 110°C erhitzt. Der beim Abkühlen ausfallende feinkristalline Niederschlag wird abgefiltert. Man erhält 140 g Substanz, die aus 700 ml Acetonitril umkristallisiert wird, wobei 115 g (0,65 Mol = 65% d.Th.) weisse Kristallnadeln mit Fp = 156 bis 158°C resultieren.

*Analyse für* $C_6H_9ClN_2O_2$: 176,6
Berechnet: Cl 20,07 N 15,86%
Gefunden: Cl 19,80 N 15,90%

$^1$H-NMR-Spektrum (in $CDCL_3$): $\delta$ = 3,5-3,8 (m,4); 5,7-6,8 (m,3); 9,05 (m,1); 10,4 (m,1)

*Beispiel 2: N-Acrylyl-N'-(2-bromäthyl)harnstoff*

Nach der Arbeitsweise von Beispiel 1 erhält man mit 165 g (1,1 Mol) 2-Bromäthylisocyanat nach 10 h bei 110°C 184 g Rohprodukt, das nach Umkristallisieren aus 600 ml Acetonitril 104 g (0,7 Mol = 70% d.Th.) weisse Kristallnadeln mit Fp = 157 bis 159°C ergibt.

*Analyse für* $C_6H_9BrN_2O_2$: 221,06
Berechnet: Br 36,15 N 12,67%
Gefunden: Br 35,90 N 12,90%

$^1H$-NMR-Spektrum (in $CDCl_3$): δ = 3,3-4,0 (m,4); 5,7-6,8 (m,3); 9,0 (m,1); 10,2 (m,1)

*Beispiel 3: N-Acrylyl-N'-(3-chlorpropyl)-harnstoff*

Nach der Arbeitsweise von Beispiel 1 erhält man mit 131,6 g (1,1 Mol) 3-Chlorpropylisocyanat nach 16 h bei 110°C 130 g Rohprodukt, das nach Umkristallisieren aus 400 ml Acetonitril 86 g (0,45 Mol = 45% d.Th.) weisse Kristallnadeln mit Fp = 105 bis 107°C ergibt.

*Analyse für* $C_7H_{11}ClN_2O_2$: 190,63

| | | |
|---|---|---|
| Berechnet: | Cl 18,60 | N 14,70% |
| Gefunden: | Cl 18,33 | N 14,49% |

$^1H$-NMR-Spektrum (in $CDCL_3$): δ = 1,75-2,3 (m,2); 3,25-3,8 (m,4); 5,7-6,8 (m,3); 8,8 (m,1); 10,55 (m,1)

*Beispiel 4: N-(2-Chloräthyl)-N'-methacrylylharnstoff*

Nach der Arbeitsweise von Beispiel 1 erhält man aus 85,1 g (1,0 Mol) Methacrylamid und 110,8 g (1,05 Mol) 2-Chloräthylisocyanat nach 10 h bei 110°C 140 g Rohprodukt, das nach Umkristallisieren aus 350 ml Acetonitril 105 g (0,55 Mol = 55% d.Th.) weisse Kristallnadeln mit Fp = 122 bis 123°C ergibt.

*Analyse für* $C_7H_{11}ClN_2O_2$: 190,63

| | | |
|---|---|---|
| Berechnet: | Cl 18,60 | N 14,70% |
| Gefunden: | Cl 18,73 | N 14,78% |

$^1H$-NMR-Spektrum (in $CDCl_3$) δ = 2,05 (d,3), 3,7 (d,4); 5,5-6,2 (m,2); 9,0 (m,1); 9,5 (m,1)

*Beispiel 5: N-Acrylyl-N'-(2-chloracetyl)harnstoff*

Zu einer Lösung von 71,1 g (1,0 Mol) Acrylamid in 150 ml 1,2-Dimethoxiäthan gibt man tropfenweise 125,5 g (1,05 Mol) 2-Chloracetylisocyanat bei 25 bis 30°C zu und rührt 24 h nach. Filtration ergibt 140 g Rohprodukt, das aus 540 ml Acetonitril umkristallisiert wird. Es resultieren 106 g (0,56 Mol = 56% d.Th.) weisse Kristalle mit Fp = 144 bis 145°C.

*Analyse für* $C_6H_7ClN_2O_3$: 190,59

| | | |
|---|---|---|
| Berechnet: | Cl 18,60 | N 14,70% |
| Gefunden: | Cl 18,70 | N 14,90% |

$^1H$-NMR-Spektrum (in $d_6$-DMSO): δ = 4,65 (s,2); 5,8-6,7 (m,3); 11,1; 11,35 (m,1)

B) Herstellung von Harnstoffen der Formel I

*Beispiel 6: Acrylylureylenäthylentrimethylammoniumchlorid*

Man kühlt 35,3 g (0,2 Mol) N-Acrylyl-N'-(2-chloräthyl)harnstoff mit 0,2 g 2,6-Di-tert.-butyl-4-methylphenol in 400 ml Aceton auf 0°C und fügt 23,6 g (0,4 Mol) flüssiges Trimethylamin zu. Man erhitzt in einem Autoklaven 20 h auf 80°C, kühlt ab und filtriert den ausgefallenen Niederschlag. Nach Waschen mit Aceton und Äther erhält man 33 g (0,14 Mol = 70% d.Th.) weisses Kristallpulver.

*Analyse für* $C_9H_{18}ClN_3O_2$: 235,71

| | | |
|---|---|---|
| Berechnet: | Cl 15,04 | N 17,83% |
| Gefunden: | Cl 14,90 | N 17,12% |

$^1H$-NMR-Spektrum (in $D_2O$): δ = 3,25 (s,9); 3,5-4,0 (m,4); 5,8-6,5 (m,3)

*Beispiel 7: Methacrylylureylenäthylentrimethylammoniumchlorid*

Nach der Arbeitsweise von Beispiel 6 erhält man aus 38,1 g (0,2 Mol) N-(2-Chloräthyl)-N'-methacrylylharnstoff 34 g (0,14 Mol = 68% d.Th.) weisses Kristallpulver.

*Analyse für* $C_{10}H_{20}ClN_3O_2$: 249,74

| | | |
|---|---|---|
| Berechnet: | Cl 14,20 | N 16,83% |
| Gefunden: | Cl 14,56 | N 16,76% |

$^1H$-NMR-Spektrum (in $D_2O$): δ = 1,95 (d,3); 3,2 (s,9); 3,5-4,0 (m,4); 5,6-5,9 (m,2)

*Beispiel 8: Acrylylureylenpropylentrimethylammoniumchlorid*

Nach der Arbeitsweise von Beispiel 6 erhält man aus 38,1 g (0,2 Mol) N-Acrylyl-N'-(3-chlorpropyl)harnstoff 47 g (0,19 Mol) = 94% d.Th.) weisses Kristallpulver, das noch Kristallaceton enthält.

*Analyse für* $C_{10}H_{20}ClN_3O_2$: 249,74

| | | |
|---|---|---|
| Berechnet: | Cl 14,20 | N 16,83% |
| Gefunden: | Cl 12,87 | N 14,59% |

$^1H$-NMR-Spektrum (in $D_2O$): δ = 1,7-2,4 (m,2); 3,15 (s,9); 3,1-3,8 (m,4); 5,8-6,7 (m,3)

*Beispiel 9: Acrylylureylenäthylentrimethylammoniumjodid*

Man kühlt 26,8 g (0,1 Mol) N-Acrylyl-N'-(2-jodäthyl)harnstoff mit 0,2 g 2,6-Di-tert.-butyl-4-methylphenol in 300 ml Aceton auf 0°C und fügt 11,8 g (0,2 Mol) flüssiges Trimethylamin zu. Man rührt 24 h und filtriert den entstandenen Niederschlag ab. Nach Waschen mit Aceton und Äther erhält man 30 g (0,092 Mol = 92% d.Th.) weisses Kristallpulver.

*Analyse für* $C_9H_{18}JN_3O_2$: 327,17

| | | |
|---|---|---|
| Berechnet: | J 38,79 | N 12,84% |
| Gefunden: | J 39,24 | N 12,55% |

$^1H$-NMR-Spektrum (in $D_2O$): δ = 3,3 (s,9); 3,6-4,0 (m,4); 5,9-6,7 (m,3)

C) Herstellung und Anwendung der Polymerisate der Formel V:

*Beispiel 10 bis 14: Copolymerisate aus Acrylamid und erfindungsgemässen Monomeren (allgemeine Vorschrift)*

Man löst das Acrylamid sowie das kationische Monomere in Wasser und stellt die Temperatur der Lösung auf 15°C und den pH-Wert auf 3,0 ein. Die Lösung wird durch Einblasen von Stickstoff von Sauerstoff befreit (1 h). Man katalysiert mit den in Tabelle 1 angegebenen Mengen 2,2'-Azo-bis-(isobutyronitril).

Tabelle 1

| Beisp. | ACA (g) | QUAT | | Wasser (g) | WS (%) | Katalysator | | | Temp. (°C) | Visk. d. 1%ig. Lösung (mPa. sec.) | Mol-Gew. vskosim. gem. |
|---|---|---|---|---|---|---|---|---|---|---|---|
| | | Menge (g) | n. Beisp. | | | AIBN (mg) | $K_2S_2O_8$ (mg) | $Na_2S_2O_4$/ $FeSO_4$ (mg) | | | |
| 10 | 90 | 10 | 6 | 300 | 25 | 5 | 3 | 10/2 | 80 | 920 | $2{,}2 \times 10^6$ |
| 11 | 70 | 30 | 6 | 300 | 25 | 10 | 6 | 20/4 | 70 | 720 | $1{,}6 \times 10^6$ |
| 12 | 90 | 10 | 7 | 300 | 25 | 5 | 3 | 10/2 | 78 | 2000 | $2{,}7 \times 10^6$ |
| 13 | 70 | 30 | 7 | 300 | 25 | 10 | 4 | 15/2 | 77 | 1040 | $1{,}5 \times 10^6$ |
| 14 | 180 | 20 | 9 | 600 | 25 | 10 | 5 | 15/2 | 70 | 500 | $1{,}8 \times 10^6$ |

ACA = Acrylamid
QUAT = quartäre (Meth)acrylylharnstoffe
WS = wirksame Substanz
AIBN = 2,2'-Azo-bis-isobutyronitril

Temp. = Temperatur
Visk. = Viskosität
gem. = Gemessen

(AIBN), Kaliumperoxodisulfat sowie Natriumdithionit und Eisen-(II)-sulfat, wonach die Polymerisation sofort anläuft. Die Maximaltemperatur wird nach etwa 30 bis 60 min erreicht. Man lässt abkühlen, zerkleinert das entstandene farblose Gel und entwässert es mit 2 bis 3 l Methanol. Nach Filtration wird anhaftendes Methanol im Vakuum abgezogen. Die Ausbeute ist etwa quantitativ.

*Beispiel 15:*

Die Anwendbarkeit der Polymerisate als Entwässerungsmittel, z.B. für Kommunalschlämme, erläutern die nachfolgend beschriebenen beiden Untersuchungen. Die Entwässerungszeiten wurden dabei nach der CST (capillary suction time)-Methode* festgestellt.

Tabelle 2

| Beispiel | Viskosität der 1%ig. Lösung i. dest. Wasser (mPa. sec.) | Kapillare Fliesszeit bei Zugabe von 0,1%ig. Lösung in Krefelder Leitungswasser ($t_F$ sec.) | | | |
|---|---|---|---|---|---|
| | | Zugabe 80 | 100 | 125 | 150 ppm |
| 10 | 920 | 71 | 45 | — | 21 |
| 11 | 720 | — | 50 | 40 | 11 |
| Produkt auf Basis **DMAEMA·$CH_3Cl$ | 9000 | — | — | 47 | 30 |
| | | Zugabe 100 | 150 | 200 | |
| 12 | 2000 | 127 | 53 | 33 | |
| 13 | 1040 | 74 | 32 | 14 | |
| Produkt auf Basis **DMAEMA·$CH_3Cl$ | 9000 | 200 | 63 | 22 | |

**DMAEMA = 2-Dimethylaminoethylmethacrylat

* Literatur: 1) R. Baskerville u. R. Gale
A simple automatic instrument for determinating the filtrability of sewage sludges
«Water pollution Control», *67* (1968), 233-241
2) R. Gale u. R. Baskerville
Capillary Suction method for determination of the filtration properties of a solid/liquid suspension
«Chemistry & Industry» (1967), 355-356

Trotz niedriger Viskosität der wässrigen Lösungen bringen die Produkte durchweg bessere Werte als die bisher angewendeten Copolymerisate auf Basis DMAEMA·$CH_3Cl$.

*Beispiel 16:*

Die Anwendbarkeit der Produkte als Entwässerungs- und Retentionshilfsmittel bei der Papierherstellung erläutern die nachfolgend beschriebenen Untersuchungen, die an standardisierten Zellstoffsuspensionen durchgeführt wurden.

Papierstoff: 60% Fichtesulfit; 40% Laubholzzellstoff

Füllstoff: 30% SPS-Clay; (Spezial-China-Clay der Firma English China Clay Co., Ltd. St.Austel, Cornwall - GB)

Mahlgrad: 38° SR

Dosierung des Hilfsmittels: 0,02% bezogen auf Papierstoff

Zur Beurteilung der Wirksamkeit der Produkte wurden die Entwässerungszeiten und der Retentionseffekt in einem Schopper-Riegler-Mahlgradprüfer ermittelt. Die Ergebnisse sind in Tabelle 3 zusammengefasst:

Tabelle 3

| Produkt | Entwässerungszeit (sec.) | Retention (%) |
|---|---|---|
| Blindprobe | 25 | 72 |
| Beispiel 10 | 21 | 90 |
| Beispiel 11 | 18 | 89 |
| Copolymerisat auf Basis DMAEMA·$CH_3Cl$ | 29 | 91 |
| DMAEMA = 2-(Dimethylamino)-ethylmethacrylat | | |

Es zeigt sich, dass die hergestellten Produkte unter den Versuchsbedingungen, die weitgehend

den Untersuchungsmethoden der Papiertechnologie entsprechen, wirksam sind. Da die Produkte in wässriger Lösung zudem leichtflüssig und daher einfach zu handhaben, d.h. leicht dosierbar und leicht löslich sind, können sie bei der Papierherstellung bevorzugt verwendet werden.

*Beispiel 17:*

Die hergestellten Produkte wurden als Sedimentationsmittel in Flockungsversuchen getestet. Durch Bestimmung der Sedimentationszeit wurde das Flockungsverhalten in wässriger Lösung nach Zusatz zu wässrigen Tonsuspensionen, die durch Aufschlämmen von Kaolin in Wasser hergestellt worden waren und mit Aluminiumsulfatlösung auf einen pH-Wert von etwa 4,8 eingestellt wurden, geprüft. Die Ergbnisse sind in Tabelle 4 dargestellt.

Tabelle 4. Flockungseffekt auf eine Tontrübe mit 20 g/l Feststoffgehalt

| Produkt | Zusatzmenge (ppm) | Zeit (sec.) |
|---|---|---|
| Blindprobe | | 180 |
| Beispiel 10 | 4 | 4,7 |
| Beispiel 11 | 4 | 14,0 |
| Beispiel 12 | 4 | 6,9 |
| Beispiel 13 | 4 | 18,6 |
| Beispiel 14 | 4 | 5,4 |

Literatur: H. Akyel und M. Neven, «Chemie-Ing. Technik», *39* (1967) 172.

**Patentansprüche für den Vertragsstaat AT**

1. Verfahren zur Herstellung von Harnstoffen der allgemeinen Formel

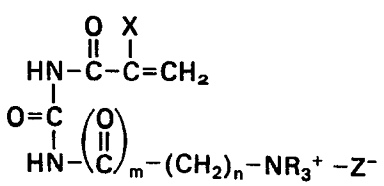
(I)

in der X entweder Wasserstoff oder eine Methylgruppe,

R ein niederer Alkylrest mit 1 bis 4 Kohlenstoffatomen ist,

m entweder 0 oder 1 und n eine ganze Zahl zwischen 1 und 4 bedeutet und Z für ein beliebiges salzbildendes Anion, vorzugsweise ein Halogenid-Ion, steht, dadurch gekennzeichnet, dass man Halogenalkyl- bzw. Halogenacylisocyanate der Formel

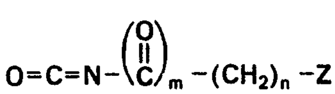
(III)

mit α,β-ungesättigten Carbonsäureamiden der Formel

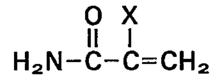
(IV)

zu halogensubstituierten Harnstoffen der allgemeinen Formel

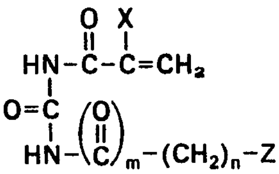
(II)

umsetzt, wobei in den vorstehenden Formen X, m und n die vorstehend angegebene Bedeutung haben und Z ein Halogenatom, vorzugsweise Chlor, Brom oder Jod ist, worauf man die halogensubstituierten Harnstoffe der allgemeinen Formel II mit einem Trialkylamin der Formel $NR_3$, in der R ein niederer Alkylrest mit 1 bis 4 Kohlenstoffatomen ist, auf an sich bekannte Weise bei Temperaturen von 20 bis 100°C und einem Druck von 1 bis 4 bar in einem Lösungsmittel zur quartären Verbindung I umsetzt.

2. Verfahren nach Anspruch 1, dadurch gekennzeichnet, dass man die Umsetzung des Isocyanats mit dem Carbonsäureamid bei Temperaturen von 20 bis 140°C und einem Druck von 1 bis 4 bar in Gegenwart eines Lösungsmittels und in Gegenwart eines tertiären Amins als Katalysator durchführt.

3. Verfahren nach Ansprüchen 1 oder 2, dadurch gekennzeichnet, dass man die Umsetzung des Isocyanats mit dem Carbonsäureamid in aromatischen Kohlenwasserstoffen oder hochsiedenden Äthern als Lösungsmittel durchführt.

4. Verfahren nach Ansprüchen 1 bis 3, dadurch gekennzeichnet, dass man die in der ersten Stufe gewonnenen halogensubstituierten Harnstoffe der Formel II mit Trimethylamin umsetzt.

5. Verfahren nach Ansprüchen 1 bis 4, dadurch gekennzeichnet, dass man die halogensubstituierten Harnstoffe der allgemeinen Formel II in Gegenwart von aliphatischen und aromatischen Kohlenwasserstoffen, Alkoholen und/oder Ketonen, vorzugsweise Aceton, als Lösungsmittel mit den Trialkylaminen umsetzt.

**Patentansprüche für die Vertragsstaaten BE, CH, DE, FR, GB, IT, LU, NL, SE**

1. Harnstoffe der allgemeinen Formel

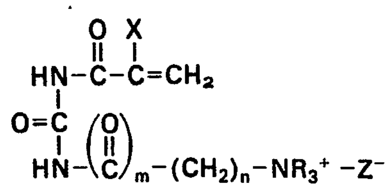
(I)

in der X entweder Wasserstoff oder eine Methylgruppe,

R ein niederer Alkylrest mit 1 bis 4 Kohlenstoffatomen ist, m entweder 0 oder 1 und n eine ganze Zahl zwischen 1 und 4 bedeutet und Z für ein beliebiges salzbildendes Anion, vorzugsweise ein Halogenidion, steht.

2. Verfahren zur Herstellung von Harnstoffen nach Anspruch 1, dadurch gekennzeichnet, dass man Halogenalkyl- bzw. Halogenacylisocyanate der Formel

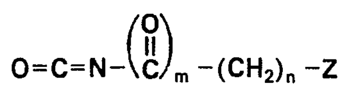
(III)

mit α,β-ungesättigten Carbonsäureamiden der Formel

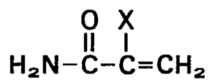
(IV)

zu halogensubstituierten Harnstoffen der allgemeinen Formel

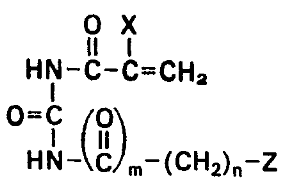
(II)

umsetzt, wobei in den vorstehenden Formeln X, m und n die in Anspruch 1 angegebene Bedeutung haben und Z ein Halogenatom, vorzugsweise Chlor, Brom oder Jod ist, worauf man die halogensubstituierten Harnstoffe der allgemeinen Formel II mit einem Trialkylamin der Formel $NR_3$, in der R ein niederer Alkylrest mit 1 bis 4 Kohlenstoffatomen ist, bei Temperaturen von 20 bis 100°C und einem Druck von 1 bis 4 bar in Abhängigkeit vom Halogensubstituenten im Ausgangsmaterial in einem Lösungsmittel zur quartären Verbindung I umsetzt.

3. Verfahren nach Anspruch 2, dadurch gekennzeichnet, dass man die Umsetzung des Isocyanats mit dem Carbonsäureamid bei Temperaturen von 20 bis 140°C und einem Druck von 1 bis 4 bar in Gegenwart eines Lösungsmittels und in Gegenwart eines tertiären Amins als Katalysator durchführt.

4. Verfahren nach Ansprüchen 2 und 3, dadurch gekennzeichnet, dass man die Umsetzung des Isocyanats mit dem Carbonsäureamid in aromatischen Kohlenwasserstoffen oder hochsiedenden Äthern als Lösungsmittel durchführt.

5. Verfahren nach Ansprüchen 2 bis 4, dadurch gekennzeichnet, dass man die in der ersten Stufe gewonnenen halogensubstituierten Harnstoffe der Formel II mit Trimethylamin umsetzt.

6. Verfahren nach Ansprüchen 2 bis 5, dadurch gekennzeichnet, dass man die halogensubstituierten Harnstoffe der allgemeinen Formel II in Gegenwart von aliphatischen und aromatischen Kohlenwasserstoffen, Alkoholen und/oder Ketonen, vorzugsweise Aceton als Lösungsmittel mit den Trialkylaminen umsetzt.

**Patent Claims for the Contracting State: AT**

1. Process for the manufacture of ureas of the general formula

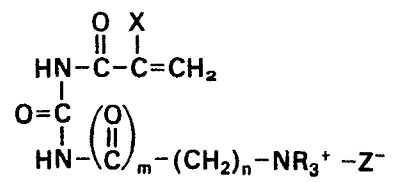
(I)

in which:

X represents either hydrogen or a methyl group,

R represents a lower alkyl radical having from 1 to 4 carbon atoms,

m represents either 0 or 1,

n represents an integer between 1 and 4, and

Z represents any salt-forming anion, preferably a halide ion,

characterised in that

haloalkyl or haloacyl isocyanates of the formula

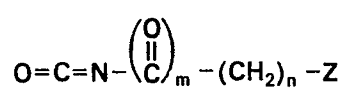
(III)

are reacted with α,β-unsaturated carboxylic acid amides of the formula

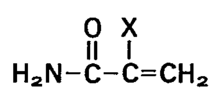
(IV)

to form halo-substituted ureas of the general formula

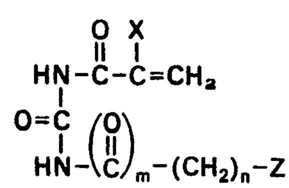
(II)

wherein, in the above formulae, X, m and n have the meanings given above and Z represents a halogen atom, preferably chlorine, bromine or iodine, whereupon the halo-substituted ureas of the general formula II are reacted with a trialkylamine of the formula $NR_3$, in which R is a lower alkyl radical having from 1 to 4 carbon atoms, in a manner known per se, at temperatures of from 20 to 100°C and at a pressure of from 1 to 4 bar and in a solvent, to form a quaternary compound I.

2. Process according to claim 1, characterised in that the reaction of the isocyanate with the carboxylic acid amide is carried out at temperatures of

from 20 to 140°C and at a pressure of from 1 to 4 bar, in the presence of a solvent and in the presence of a tertiary amine as catalyst.

3. Process according to claim 1 or 2, characterised in that the reaction of the isocyanate with the carboxylic acid amide is carried out in aromatic hydrocarbons or high-boiling ethers as solvent.

4. Process according to claims 1 to 3, characterised in that the halo-substituted ureas of the formula II obtained in the first stage are reacted with trimethylamine.

5. Process according to claims 1 to 4, characterised in that the halo-substituted ureas of the general formula II are reacted with the trialkylamines in the presence of aliphatic and aromatic hydrocarbons, alcohols and/or ketones, preferably acetone, as solvent.

**Patent Claims for the Contracting States: BE, CH, DE, FR, GB, IT, LU, NL, SE**

1. Ureas of the general formula

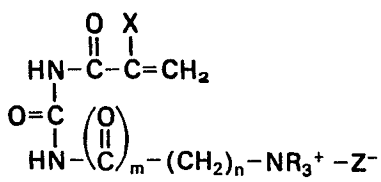
(I)

in which:

X represents either hydrogen or a methyl group,

R represents a lower alkyl radical having from 1 to 4 carbon atoms,

m represents either 0 or 1,

n represents an integer between 1 and 4, and

Z represents any salt-forming anion, preferably a halide ion.

2. Process for the manufacture of ureas according to claim 1, characterised in that haloalkyl or haloacyl isocyanates of the formula

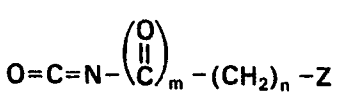
(III)

are reacted with α,β-unsaturated carboxylic acid amides of the formula

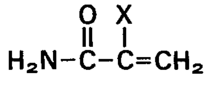
(IV)

to form halo-substituted ureas of the general formula

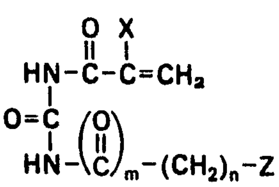
(II)

wherein, in the above formulae, X, m and n have the meanings given in claim 1 and Z represents a halogen atom, preferably chlorine, bromine or iodine, whereupon the halo-substituted ureas of the general formula II are reacted with a trialkylamine of the formula $NR_3$, in which R is a lower alkyl radical having from 1 to 4 carbon atoms, at temperatures of from 20 to 100°C and at a pressure of from 1 to 4 bar depending upon the halogen substituents in the starting material, and in a solvent, to form a quaternary compound I.

3. Process according to claim 2, characterised in that the reaction of the isocyanate with the carboxylic acid amide is carried out at temperatures of from 20 to 140°C and at a pressure of from 1 to 4 bar, in the presence of a solvent and in the presence of a tertiary amine as catalyst.

4. Process according to claims 2 and 3, characterised in that the reaction of the isocyanate with the carboxylic acid amide is carried out in aromatic hydrocarbons or high-boiling ethers as solvent.

5. Process according to claims 2 to 4, characterised in that the halo-substituted ureas of the formula II obtained in the first stage are reacted with trimethylamine.

6. Process according to claims 2 to 5, characterised in that the halo-substituted ureas of the general formula II are reacted with the trialkylamines in the presence of aliphatic and aromatic hydrocarbons, alcohols and/or ketones, preferably acetone, as solvent.

**Revendications pour l'Etat contractant: AT**

1. Procédé de préparation de dérivés d'urée de la formule générale

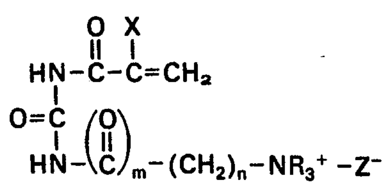
(I)

dans laquelle X désigne un atome d'hydrogène ou un radical méthyle, R un radical alcoyle inférieur en $C_1$ à $C_4$, m vaut 0 ou 1, n est un nombre entier entre 1 et 4 et Z représente un anion salifiant quelconque, de préférence un ion halogénure, caractérisé en ce que l'on fait réagir un isocyanate d'haloalcoyle ou un isocyanate d'haloacyle de la formule

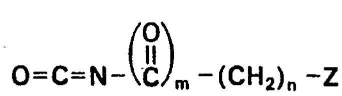
(III)

avec un amide d'acide carboxylique à insaturation α,β de la formule

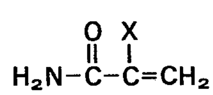
(IV)

dans lesquelles formules X, m et n possèdent la signification définie plus haut et Z désigne un atome d'halogène et de préférence un atome de chlore, de brome ou d'iode, puis le dérivé d'urée halosubstitué formé, de la formule générale

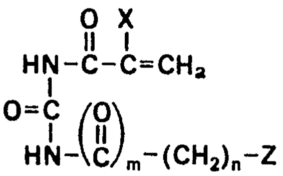

(II)

avec une trialcoylamine de la formule $NR_3$, où R désigne un radical alcoyle inférieur en $C_1$ à $C_4$, de manière connue, dans un solvant à des températures entre 20 et 100°C et sous une pression de 1 à 4 bars pour l'obtention d'un composé quaternaire de la formule I.

2. Procédé suivant la revendication 1, caractérisé en ce que la réaction de l'isocyanate et de l'amide carboxylique est réalisée dans un solvant à des températures de 20 à 140°C et sous une pression de 1 à 4 bars en présence d'une amine tertiaire comme catalyseur.

3. Procédé suivant l'une des revendications 1 et 2, caractérisé en ce que la réaction de l'isocyanate et de l'amide carboxylique est réalisée dans un solvant choisi parmi les hydrocarbures aromatiques et les éthers à point d'ébullition élevé.

4. Procédé suivant les revendications 1 à 3, caractérisé en ce que l'on fait réagir le dérivé d'urée halosubstitué de la formule II, obtenu dans le premier stade, avec la triméthylamine.

5. Procédé suivant les revendications 1 à 4, caractérisé en ce que la réaction des dérivés d'urée halosubstitués de la formule générale II avec les trialcoylamines est effectuée dans un solvant choisi parmi les hydrocarbures aliphatiques et aromatiques, les alcools et (ou) les cétones, le solvant préféré étant l'acétone.

**Revendications pour les Etats contractants: BE, CH, DE, FR, GB, IT, LU, NL, SE**

1. Dérivés d'urée de la formule générale

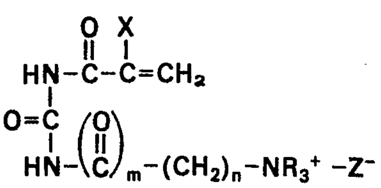

(I)

dans laquelle X désigne un atome d'hydrogène ou un radical méthyle, R un radical alcoyle inférieur en $C_1$ à $C_4$, m vaut 0 ou 1, n est un nombre entier entre 1 et 4 et Z représente un anion salifiant quelconque, de préférence un ion halogénure.

2. Procédé de préparation de dérivés d'urée suivant la revendication 1, caractérisé en ce que l'on fait réagir un isocyanate d'haloalcoyle ou un isocyanate d'haloacyle de la formule

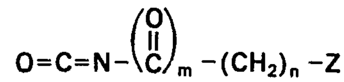

(III)

avec un amide d'acide carboxylique à insaturation α,β de la formule

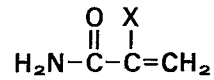

(IV)

dans lesquelles X, m et n possèdent les significations définies dans la revendication 1 et Z désigne un atome d'halogène, de préférence un atome de chlore, de brome ou d'iode, puis le dérivé d'urée halo-substitué formé, de la formule générale

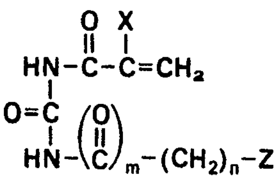

(II)

dans laquelle X, Z, m et n possèdent les significations définies, avec une trialcoylamine de la formule $NR_3$, où R désigne un radical alcoyle inférieur en $C_1$ à $C_4$, dans un solvant à des températures entre 20 et 100°C et sous une pression de 1 à 4 bars, qui est fonction du substituant halogéné du composé de départ, pour l'obtention d'un composé quaternaire I.

3. Procédé suivant la revendication 2, caractérisé en ce que la réaction de l'isocyanate et de l'amide carboxylique est réalisée dans un solvant à des températures entre 20 et 140°C et sous une pression de 1 à 4 bars en présence d'une amine tertiaire comme catalyseur.

4. Procédé suivant l'une des revendications 2 et 3, caractérisé en ce que le solvant pour la réaction de l'isocyanate et de l'amide carboxylique est choisi parmi les hydrocarbures aromatiques et les éthers à point d'ébullition élevé.

5. Procédé suivant l'une des revendications 2 à 4, caractérisé en ce que l'on fait réagir le dérivé d'urée halosubstitué de la formule II, obtenu dans le premier stade, avec la triméthylamine.

6. Procédé suivant l'une des revendications 2 à 5, caractérisé en ce que la réaction du dérivé d'urée halosubstitué de la formule II avec une trialcoylamine est effectuée dans un solvant choisi parmi les hydrocarbures aliphatiques et aromatiques, les alcools et(ou) les cétones, le solvant préféré étant l'acétone.